# EUROPEAN PATENT SPECIFICATION

(11) **EP 0 505 686 B1**
(45) Date of publication and mention of the grant of the patent: **13.11.1996**
(21) Application number: 92101355.3
(22) Date of filing: 28.01.1992
(51) Int. Cl.: A61F 2/06

(54) **Stent delivery system**
System zum Einführen eines Gefässerweiterungsgerätes
Système de délivrance pour dilateur de vaisseaux

(30) Priority: 28.01.1991 US 647464
(43) Date of publication of application: 30.09.1992
(73) Proprietor: ADVANCED CARDIOVASCULAR SYSTEMS, INC., Santa Clara California 95052 (US)
(72) Inventor: Lau, Lilip, Cupertino, California 95014 (US); Hartigan, William M., Fremont, California 94555 (US)
(74) Representative: Molyneaux, Martyn William

(56) References cited:
- EP-A- 0 408 245
- WO-A-89/01798
- WO-A-89/08433
- DE-A- 3 640 745
- US-A- 4 748 982

## Description

### BACKGROUND OF THE INVENTION

This invention relates to devices for the treatment of heart disease and particularly to a system allowing the rapid delivery through a patient's body lumen of endoarterial prostheses, which commonly are called stents as specified in the preamble of Claim 1. Such a system is known e.g. from DE-A-3 640 745.

Several interventional treatment modalities presently are used for heart disease, which include balloon and laser angioplasty, atherectomy and by-pass surgery.

In typical balloon angioplasty procedures a guiding catheter having a preformed distal tip is introduced percutaneously, through the femoral artery, into the cardiovascular system until the distal tip of the guiding catheter is seated in the ostium of a coronary artery in which treatment is to be rendered. A guidewire is positioned within an inner lumen of a dilatation catheter, and then both are advanced through the guiding catheter to the distal end thereof. The guidewire is first advanced out of the distal end of the guiding catheter into the coronary vasculature of the patient until the distal end of the guidewire crosses a lesion to be dilated, then the dilatation catheter having an inflatable balloon on the distal portion thereof is advanced into the patient's coronary anatomy, over the previously introduced guidewire, until the balloon of the dilatation catheter is properly positioned across the lesion. Once in position across the lesion, the balloon, which is made of relatively inelastic materials, is inflated to a predetermined size with radiopaque liquid at relatively high pressures (*e.g*., greater than 4.05 X 10⁵ pascals (4 atmospheres)) to compress the arteriosclerotic plaque of the lesion against the inside of the artery wall and otherwise to expand the inner lumen of the artery. The balloon then is deflated so that blood flow can resume through the dilated artery and the dilatation catheter can be removed therefrom. Often more than one balloon dilatation of a targeted area of stenosis must be performed in order to result in an angioplasty procedure being deemed successful. Accordingly, systems have been developed to allow one balloon dilatation catheter to be exchanged for another while leaving the previously-introduced guidewire still in place. Such systems include those in which the length of the guidewire effectively is extended outside the patient's body, so that the dilatation catheter can be fully withdrawn over the guidewire while yet allowing the guidewire continuously to be held in position throughout the removal process. Two of this type of system are disclosed in US-A-4,827,941 and GB 2 180 454A. In other dilatation catheter exchange systems, the guidewire can be threaded through two ports in a catheter over a distance that is less than the entire length of the catheter, but still long enough to maintain sufficient torqueability and pushability of the guidewire in the catheter. One such system is disclosed in U.S. Patent 4,748,982 (Horzewski et al.). Further details of dilatation catheters, guidewires, and devices associate therewith for angioplasty procedures can be found in U.S. Patent 4,323,071 (Simpson-Robert); U.S. Patent 4,439,185 (Lundquist); U.S. Patent 4,516,972 (Samson); U.S. Patent 4,538,622 (Samson *et al*.); U.S. Patent 4,554,929 (Samson *et al*.); U.S. Patent 4,616,652 (Simpson); U.S. Patent 4,638,805 (Powell).

A problem which can occur during balloon angioplasty procedures is the formation of intimal flaps, which can collapse and occlude the artery after the balloon is delated at the end of the angioplasty procedure. Another problem after a first balloon angioplasty procedure has been performed is that a large number of patients experience restenosis of the treated artery. In the case of restenosis, the treated artery may again be subjected to balloon angioplasty or to other treatments such as by-pass surgery, if further balloon angioplasty is not warranted. However, in the event that a coronary artery is either partially or totally occluded by the collapse of a dissected arterial lining after a balloon is deflated, the patient is put in an extremely dangerous situation requiring immediate medical attention, particularly when the occlusion is present in a coronary artery.

A primary focus of recent development work in the treatment of coronary artery disease has been directed to endoprosthetic devices called stents. Stents are somewhat akin to graft prostheses which commonly are used to treat aneurysms occurring in areas of major vessels such as the abdominal aorta. A delivery system for such grafts is disclosed in WO 89/08433, in which a catheter tube bearing "pusher buttons" is manipulated to urge a graft out of a protective capsule whereupon it is anchored to the vessel walls by means of staples or the like. Another delivery system is disclosed in DE 3 640 745 in which a stent is mounted on a balloon catheter, and a sheath covers both the balloon catheter and the stent while delivering the stent to a treatment site in a vessel.

In contrast to grafts of the type disclosed in WO 89/108433, however, stents typically are indicated as a form of treatment immediately after some other intravascular procedure has been performed, such as balloon angioplasty of stenosed vessels. Generally, stents are cylindrically-shaped intravascular devices which are placed within a damaged artery to hold it upon, thus discouraging restenosis of the previously treated are and maintaining the patency of the vessel. In some circumstances, a stent, like a graft, can be used as a primary treatment device, when the device is expanded to dilate an artery.

Generally stents are cylindrically-shaped intravascular devices which are placed within a damaged artery to hold it open. Such a device can be used to discourage restenosis and to maintain the patency of the blood vessel immediately after intravascular treatments.

However, the physician's ability to effectively and rapidly deliver a stent to the desired location within the vasculature of the patient has been less than optimum with the delivery systems heretofore known particularly in those circumstances in which an intimal flap occludes an artery. Attempts to advance a stent into regions of coronary arteries occluded by dissected arterial linings have not been very successful.

Two basic systems have been developed for less temporary deployment of stents to desired locations within body lumens. One system involves compressing or otherwise reducing the diameter of an expandable stent, disposing the compressed stent within a lumen provided in the distal end of a tubular catheter, advancing the catheter through the vasculature of a patient until the distal end of the catheter is immediately adjacent the desired vascular location, and then pushing the stent out of the distal end of the catheter into the desired location. Once out of the catheter, the compressed stent expands or is expanded by suitable means, to thereby hold open the artery or other body lumen into which it has been placed.

Another system involves disposing a compressed or otherwise small diameter stent about an expandable member, such as a balloon, on the distal end of a catheter, advancing the catheter through the patient's vascular system until the stent is in the desired location within a blood vessel, and then expanding the expandable member on the catheter to deploy the stent within the blood vessel. The expanded expandable member then is contracted and the delivery catheter is withdrawn, leaving the expanded stent within the blood vessel to maintain the patency thereof.

The following references illustrate various types of stents and stent delivery systems the list is meant to be exemplary not exhaustive on the subject: U.S. 3,868,956; U.S. 4,503,569; U.S. 4,512,338; U.S. 4,553,545; U.S. 4,560,374; U.S. 4,655,771; U.S. 4,665,918; U.S. 4,733,665; U.S. 4760,849; U.S. 4,762,128; U.S. 4,768,507; U.S. 4,795,458; U.S. 4,800,882; U.S. 4,830,003; U.S. 4,856,516; U.S. 4,878,906; U.S. 4,886,062; U.S. 4,907,336; U.S. 4,913,141; U.S. 4,923,464; and U.S. 4,950,227.

What has been needed and heretofore unavailable is a stent delivery system which can be quickly and easily used in a wide variety of situations, and particularly in emergency situations, when a dissected arterial lining has collapsed and has occluded the flow of blood to a vital organ. The present invention satisfies this need.

### SUMMARY OF THE INVENTION

This invention is directed to an improved stent delivery system allowing the rapid delivery through a patient's body lumen of an expandable stent as specified in Claim 1.

The first inner lumen includes a sheath sidewall port cut out of the sheath at a distance proximal of the sheath end port, the sheath sidewall port being in fluid communication with the first inner lumen. The guidewire lumen includes a catheter sidewall port cut out thereof at a distance proximally of the expandable member, the catheter sidewall port being in fluid communication with both the guidewire inner lumen and the sheath inner lumen, the ports in the catheter and the sidewall port in the sheath cooperating to allow the delivery system to be rapidly advanced over the in-place guidewire.

Preferably, both the delivery sheath and the intravascular catheter have slits in the walls thereof, which extend distally from the proximal port of each, to facilitate the disengagement of these devices from the guidewire when withdrawal of the delivery system is desired after a stent has been situated in the vascular system of a patent.

In the course of a typical angioplasty procedure, the guidewire used to delivery a dilatation catheter through the patient's vascular system to a stenotic region, is left disposed within the patient after the dilatation catheter has been removed. To preserve the ability to maintain access to the stenotic region with other, subsequently-deployed treatment devices, the distal end of the guidewire commonly is left crossing the stenotic region where a stent might be required to be placed. To introduce into a patient a stent delivery system in accordance with the invention, the proximal end of the guidewire, *e.g*., the end that extends out of the patient, is inserted through the first port in the distal end of the intravascular catheter, which catheter at this point has a stent mounted on the expandable member thereof. The intravascular catheter is disposed within the inner lumen of the delivery sheath, and the distal end of it extends out of the port in the distal end of the delivery sheath, to facilitate the insertion of the proximal end of the guidewire. The relative axial position of the delivery sheath and of the intravascular catheter are adjusted so that the expandable member on the distal extremity of the catheter having the expandable stent mounted thereon, is caused to be contained within the inner lumen of the delivery sheath. The delivery sheath and the catheter then are advanced over the guidewire through the vascular system of the patient desirably through a previously-inserted guiding catheter, which extends from outside the patient to the ostium of the desired coronary artery, until the stent mounted on the expandable member of the intravascular catheter is positioned within the region of the patient's blood vessel in which treatment is to be rendered.

The relative axial positions of the delivery sheath and the intravascular catheter carrying the stent, then are adjusted so as to urge the distal end of the catheter out of the distal end of the sheath to expose the expandable stent. To accomplish this, either the catheter can be advanced distally with respect to the sheath, or the sheath can be withdrawn proximally with respect to the catheter alternatively, a combination of both of these movements can be employed. When the stent is completely out of the delivery sheath and exposed to the site at which treatment is to be rendered, the expandable member on the catheter can be caused to expand to deploy the stent against stenotic mass. After the stent has been deployed, the expandable member is contracted or deflated to allow so the catheter to be removed from the patient, leaving behind the now fully deployed stent.

When the stent has been delivered, the delivery sheath and the intravascular catheter can be withdrawn in unison, or the sheath can be withdrawn first, followed by removal of the catheter. Both are withdrawn over the guidewire until the proximal or second guidewire port of the sheath and/or of the catheter exits the proximal end of the guiding catheter. Thereupon, the sheath and the catheter effectively can be peeled away from the guidewire, the guidewire sliding through the slits which extend distally from each of the proximal ports. The sheath and the intravascular catheter are pulled proximally out of the proximal end of the guiding catheter a sufficient distance to expose the guidewire. The exposed section of the guidewire is secured, *e.g*., by being held in position manually, so that the sheath and the intravascular catheter can be removed from the patient without dislodging the position of the guidewire in the patient's vasculature.

Thus, the delivery system of the invention can effectively deliver a stent to a desired location within a patient's blood vessel, and allow a stent to be secured at the location at which treatment is to be rendered, and then easily and quickly can be removed. These and other advantages of the invention will become more apparent from the following detailed description of the invention, when taken in conjunction with the accompanying exemplary drawings.

### BRIEF DESCRIPTION OF THE DRAWINGS

Fig. 1 is a partial longitudinal cross-sectional view of a stent delivery system which embodies features of the invention.

Fig. 2 is a top view of the delivery sheath shown in Fig. 1.

Fig. 3 is a transverse cross-sectional view taken along the lines 3-3 shown in Fig. 1.

Fig. 4 is a transverse cross-sectional view taken along the lines 4-4 shown in Fig. 1.

Fig. 5 illustrates a stent mounted on the outer surface of a balloon of the dilatation catheter shown in Fig. 1.

Fig. 6 illustrates the advancement of the stent delivery system shown in Fig. 5 into an artery which has been damaged by an intravascular procedure such as an angioplasty.

Fig. 7 illustrates the inflation of the balloon on the dilatation catheter shown in Fig. 1 which expands the stent mounted on the exterior thereof.

Fig. 8 illustrates the expanded stent disposed within a damaged arterial section maintaining the patency thereof.

Fig. 9 is a partial cross-sectional view of the manipulator shown in Fig. 1.

Fig. 10 is a perspective view of an alternative manipulator mounted on the proximal end of the delivery system shown in Fig. 1.

Fig. 11 is a plan view of the manipulator shown in Fig. 10.

Fig. 12 is an elevational view, partially in section, of the manipulator shown in Fig. 10.

### DETAILED DESCRIPTION OF THE INVENTION

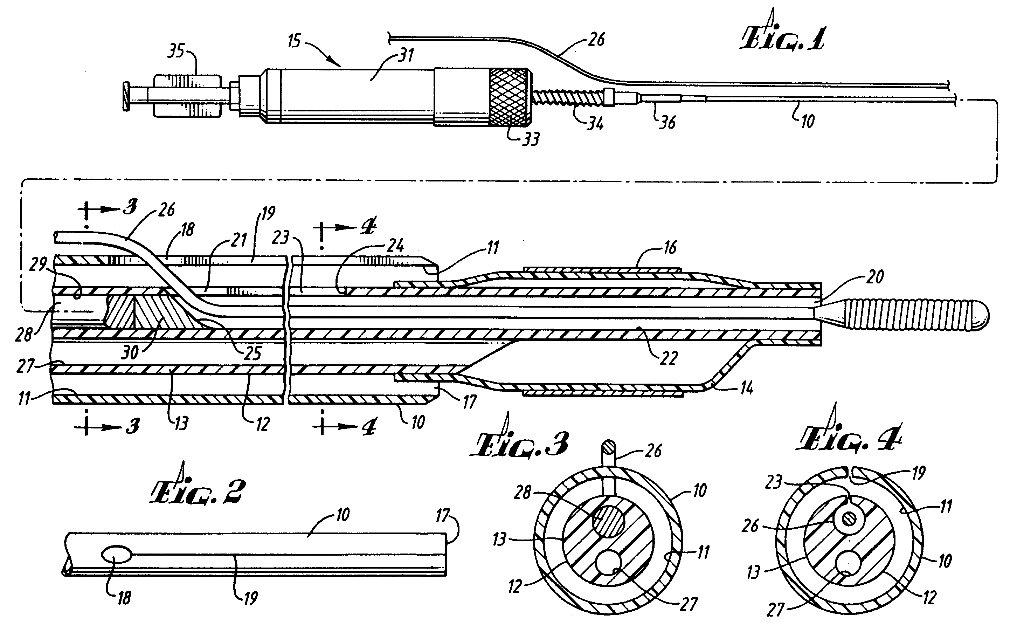

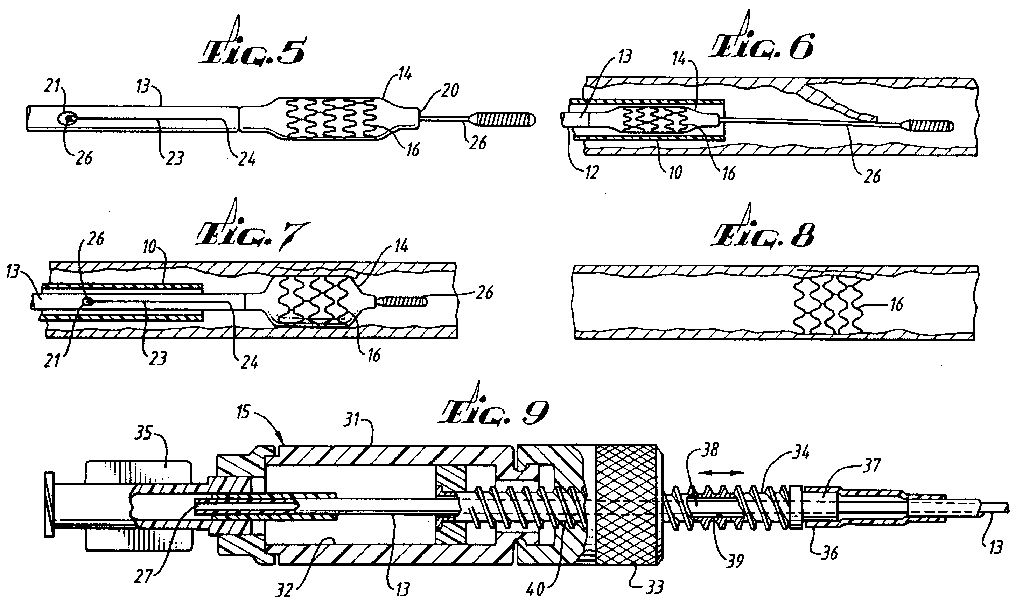

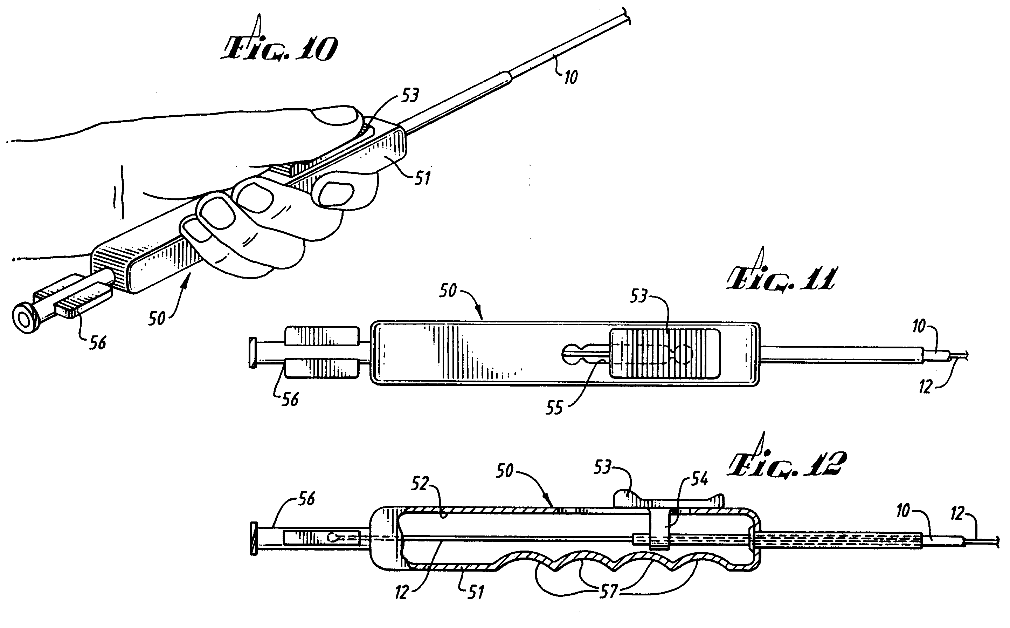

Figs. 1-4 illustrate a stent delivery system which embodies features of the invention. Generally, the delivery system includes a delivery sheath 10 which has an inner lumen 11 and a dilatation catheter 12 disposed within the inner lumen 11 which has an elongated catheter body 13 and a balloon 14 on the distal portion of the catheter body. A manipulating device 15 is provided on the distal end of the delivery system which is employed to effect relative axial or longitudinal movement between the delivery sheath 10 and the dilatation catheter 12. An expandable stent 16, which is to be delivered within a patient's body lumen, is mounted on the exterior of the balloon 14.

The delivery sheath 10 has a distal port 17 in its distal end which is in fluid communication with the inner lumen 11 and a proximal port 18 disposed proximally to the distal port. A slit 19 extends from the proximal port 18 to a location just proximal to the distal port 17.

The dilatation catheter 12 has a distal port 20 and a proximal port 21 which are in fluid communication with a first inner lumen 22 extending within the distal portion of the catheter 12 and being adapted to slidably receive a guidewire therein. A slit 23 extends from the proximal port 21 to a location 24 proximal to the proximal end of balloon 14. The proximal end of the guidewire receiving lumen 22 is provided with a ramp 25 to guide the proximal end of guidewire 26 out the proximal port 21 in the catheter 12 when the catheter is mounted onto the guidewire as will be discussed hereinafter. A second, much longer inner lumen 27 is provided within the catheter body 13 to direct inflation fluid from the proximal end of the catheter body to the interior of the balloon 14.

Proximal to the proximal port 21 in the catheter body 13 is a stiffening member 28 which is disposed in third inner lumen 29 provided within the catheter body 13. As shown in the drawings, the third inner lumen 29 and the first inner lumen 22 may be the same lumen with a plug 30 separating the two lumens. The ramp 25 is on the distal side of the plug 30.

As illustrated in Figs. 1 and 9, the manipulator 15 on the proximal end of the delivery system has a housing 31 with an interior chamber 32, a cap 33 rotatably mounted onto the distal end of the housing 31, an elongated drive member 34 which has male threads on the exterior thereof and which is at least partially disposed within the interior chamber 32 and a Luer™ lock 35 which is fixed within the proximal end of the housing 31. The proximal end 36 of the sheath 10 is secured to the distal end 37 of the elongated drive member 34 which extends out of the distal end of the housing 31. As shown in more detail in Fig. 9, the proximal end 38 of the catheter body 13 passes through passageway 39 in the elongated drive member 34 and is fixed within the Luer™ lock 35 by suitable means such as adhesive. The cap 33 which is rotatably mounted onto the distal end of the housing 31 is provided with an inner threaded collar 40 adapted to threadably engage the threaded exterior of the elongated driving member 34. Rotation of the cap 33 axially moves the driving member 34 to thereby effect relative axial movement between the sheath 10 and the dilatation catheter 12.

In a typical situation, the stent delivery system of the invention is used after a intravascular procedure has damaged a patient's arterial lining to such a extent that the lining needs support to prevent it from collapsing into the arterial passageway and thereby preventing sufficient blood flow through the blood vessel. In these situations there will usually be a guidewire 26 (or other guiding member) in place extending across the damaged section of the artery such as shown in Fig. 6. The proximal end of the guidewire 26, which extends out of the patient during the entire procedure, is inserted through the distal port 20 in the distal end of the catheter 12 and advanced proximally through the first inner lumen 22 until the proximal end of the guidewire impacts the ramp 25 and is thereby directed through the proximal port 21.

The dilatation catheter 12 is preferably positioned within the inner lumen 11 of the delivery sheath 10 so that at least a significant portion of the proximal port 18 in the sheath is in alignment with the proximal port 21 of the dilatation catheter. In this manner, proximal advancement of the guidewire 26 through the inner lumen 22 will also direct the proximal end of the guidewire out the proximal port 18 in the delivery sheath 10. The proximal end of the guidewire 26 may then be manually held to maintain the position of the guidewire within the patient's vasculature, while the stent delivery system is advanced over the guidewire system. The advancement of the stent delivery system continues until the distal ends of the catheter and sheath extend adjacent to or across the damaged arterial site. At this point in the procedure, the manipulator 15 on the proximal end of the delivery system is actuated by rotating the cap 33 on the proximal end of the housing 31 to move the sheath 10 proximally with respect to the catheter 12 and thereby expose the stent 16 mounted on the balloon 14. When the balloon and the stent mounted thereon are properly placed within the damaged artery, inflation fluid is directed under substantial pressure through the Luer™ lock 35 and the inflation lumen 27 in the catheter body 13 to the interior of the balloon 14, expanding the balloon and simultaneously expanding the stent 16 against the blood vessel wall as shown in Fig. 7. The delivery system, both the sheath 10 and the catheter 12, may then be removed from the patient along with the guidewire 26, leaving the expanded stent 16 within the damaged arterial section as shown in Fig. 8 to maintain the patency thereof.

The housing 31 of the manipulator 15 can be held in the palm of the physician's hand, with the thumb and index finger thereof used to rotate cap 33 and thereby cause the necessary relative motion between the sheath 10 and dilatation catheter 12 to expose the stent 16 mounted on the balloon 14. The physician can operate an inflation device, such as described in U.S.Patent 4,439,185, with his or her free hand to inject inflation fluid through Luer™ lock 35 into the interior of the balloon 14 to inflate the balloon and thereby expand the stent 16 while holding the delivery system in place with the other hand. Upon deflating the balloon 14, the manipulator 15 can again be actuated by the physician rotating cap 33 with the fingers of the hand holding the manipulator 15 to pull the dilatation catheter 12 back into the distal end of the sheath 10 (or pushing the distal end of the sheath over the distal end of the dilatation catheter 12, depending upon the perspective) and then the entire assembly, including the guidewire 26 can be removed from the patient.

The alternative manipulator 50 illustrated in Figs. 10-12 generally includes a housing 51 with an interior chamber 52 and a slidable element 53 with a depending portion 54 which extends through a slot 55 in the wall of the housing and is secured to the proximal end of the sheath 10 which extends through an opening provided in the distal end of the housing. The catheter 12 extends out the proximal end of the sheath 10, out an opening in the proximal end of the housing 51 and into a Luer™ lock 56 secured to the proximal end of the housing. The proximal end of the catheter 12 is secured within the Luer™ lock 56 to be in fluid communication with the inner inflation lumen 27 of the catheter so that inflation fluid can be injected through the Luer™ lock to the interior of the balloon 14 on the catheter to expand the balloon and the stent 16 mounted thereon. As is evident from Fig. 10, movement of element 53 on the exterior of the housing 51 will effect the relative axial movement between the delivery sheath 10 and the catheter 12 required to expose the stent 16 mounted on the balloon 14. The slot 55 has narrowed portions near both ends thereof which have widths just lightly smaller than the depending element 54 so that the position of the slidable element 53 can be locked.The underside of the housing 51 may be provided with an undulated surface 57 which is adapted to receive the fingers of an operator to facilitate the gripping thereof.

The dimensions of the dilatation catheter will generally follow the dimensions of dilatation catheters used in angioplasty procedures in the same arterial location. Typically, the length of a catheter for use in the coronary arteries is about 150 cm, the outer diameter of the catheter shaft is about 0.035 inch (0.89 mm), the length of the balloon is typically about 2 cm and the inflated diameter about 1 to about 8 mm.

The materials of construction may be selected from those used in conventional balloon angioplasty catheters, such as those described in the patents incorporated by reference. The delivery sheath will generally be slightly shorter than the dilation catheter (*e.g*., by about the length of the manipulating device 15 or 50, with an inner diameter large enough to accommodate the dilation catheter and allow the catheter free longitudinal movement therein). The sheath and the catheter shaft can be made of conventional polyethylene tubing.

## Claims

1. A system allowing the rapid delivery through a patient's body lumen of an expandable stent over an in-place guidewire (26), the system having an elongated sheath (10) with proximal and distal ends, which protects the stent during delivery and which is remotely removable from the stent to allow deployment of the same; a first inner lumen (11) extending between the proximal end and the distal end of the sheath (10), the lumen and sheath defining a sheath end port (17) in the distal end, the port (17) being in fluid communication with the first inner lumen (11); an elongated catheter (12) with proximal and distal ends, disposed within the first inner lumen (11) of the sheath (10), the catheter having a expandable member (14) proximally adjacent to its distal end, the expandable member (14) being adapted to receive about the exterior thereof an expandable stent (16), the catheter further having an inflation inner lumen (27) in fluid communication with the interior of the expandable member (14), and a guidewire inner lumen (22) extending between a point proximal of the proximal end of the expandable member to the distal end of the catheter (12), the guidewire lumen (22) and the distal end of the catheter defining a catheter end port (20) in fluid communication with both the guidewire inner lumen (22) and the sheath inner lumen (11); and means (15) to adjust the axial position of the sheath (10) with respect to the position of the catheter (12), operable remotely from the patient, the adjustment means allowing the stent (16) to be disposed on the expandable member (14) to be exposed to the interior of the body lumen, at which point the stent can be expanded and deployed by inflation of the expandable member; the system being characterized in that:
the lumen (11) includes a sheath sidewall port (18) cut out of the sheath at a distance proximal of the sheath end port, the sheath sidewall port (18) being in fluid communication with the first inner lumen (11); and
the guidewire lumen (22) includes a catheter sidewall port (21) cut out thereof at a distance proximally of the expandable member, the catheter sidewall port (21) being in fluid communication with both the guidewire inner lumen (22) and the sheath inner lumen (11), the catheter sidewall port (21) in the catheter and tile sheath sidewall port (18) in the sheath (10) cooperating to allow the delivery system to be rapidly advanced over the in-place guidewire (26).

2. The system of claim 1, further characterized by a slit (19) which is disposed between the sheath distal end port (17) and the sheath sidewall port (18) so that the sheath (10) can be moved axially and proximally relative to the catheter (12) without changing the position of the guidewire (26), so as to expose the stent (16) on the expandable member (14) to the interior of the body lumen for deployment.

3. The system of claims 1 or 2, further characterized by a slit (23) is disposed between the catheter end port (20) and the catheter sidewall port (21).

4. The system of any one of the preceding claims, further characterized in that the adjustment means includes a manipulator (15) comprising:
an elongated housing (31) having proximal and distal ends and an interior chamber;
a cap (33) having a threaded passageway therethrough and which is rotatably mounted on an end of the elongated housing (31); and
a longitudinally moveable drive member (34) having a threaded exterior, which is disposed at least partially within the interior chamber of the elongated housing, and further having a distal end extending through the central passageway of the cap (33), rotation of the cap causing movement of the axial movement of the drive member (34).

5. The system of claim 4, further characterized in that the longitudinally movable drive member (34) has a central passageway adapted to receive the proximal end of the catheter (12).

6. The system of claim 5, further characterized in that the proximal end of the catheter (12) is fixed to the manipulator housing (31).

7. The system of claims 5 or 6, further characterized in that the sheath (10) is fixed to the distal end of the longitudinally movable drive member (34) extending out of the distal end of the manipulator (15).

8. The system of any one of the preceding claims, further characterized by an expandable stent (16) mounted about the exterior of the expandable member (14).

## Patentansprüche

1. System, das eine schnelle Durchführung einer dehnbaren Streckeinrichtung über einen sich an der Verwendungsposition befindenden Führungsdraht (26) durch ein Lumen eines Patientenkörpers ermöglicht, wobei das System eine elongierte Hülle (10) mit proximalen und distalen Enden aufweist, die die Streckeinrichtung während der Durchführung schützt und die aus der Ferne von der Streckeinrichtung entfernt werden kann, um eine Entfaltung dieser zu ermöglichen; mit einem ersten inneren Lumen (11), das sich zwischen dem proximalen und dem distalen Ende der Hülle (10) erstreckt, wobei das Lumen und die Hülle in dem distalen Ende eine Hüllenendenöffnung (17) begrenzen, wobei sich die Öffnung (17) in Fluidverbindung mit dem ersten inneren Lumen (11) befindet; mit einem elongierten Katheter (12) mit proximalen und distalen Enden, der sich in dem ersten inneren Lumen (11) der Hülle (10) befindet, wobei der Katheter proximal neben dessen distalen Ende ein dehnbares Element (14) aufweist, wobei das dehnbare Element (14) um das Äußere des Elements eine dehnbare Streckeinrichtung (16) aufnehmen kann, wobei der Katheter ferner ein inneres Inflationslumen (27) aufweist, das sich in Fluidverbindung mit dem Inneren des dehnbaren Elements (14) befindet, und wobei sich ein inneres Führungsdrahtlumen (22) zwischen einer zu dem proximalen Ende des dehnbaren Elements proximalen Stelle und dem distalen Ende des Katheters (12) erstreckt, wobei das Führungsdrahtlumen (22) und das distale Ende des Katheters eine Katheterendöffnung (20) begrenzen, die sich in Fluidverbindung mit dem inneren Führungsdrahtlumen (22) und dem inneren Hüllenlumen (11) befindet; und mit einer Einrichtung (15) zur Einstellung der axialen Position der Hülle (10) im Verhältnis zu der Position des Katheters (12), wobei die Einrichtung entfernt von dem Patienten bedienbar ist, wobei es die Einstellungseinrichtung ermöglicht, daß die Streckeinrichtung (16) an dem dehnbaren Element (14) angeordnet werden kann, so daß sie zu dem Inneren des Körperlumens ausgerichtet ist, wobei die Streckeinrichtung an dieser Stelle gedehnt und durch Inflation des dehnbaren Elements entfaltet werden kann, wobei das System dadurch gekennzeichnet ist, daß:
das Lumen (11) eine Hüllenseitenwandöffnung (18) aufweist, die ein Stück von der Hüllenendenöffnung entfernt aus der Hülle ausgeschnitten ist, wobei sich die Hüllenseitenwandöffnung (18) in Fluidverbindung mit dem ersten inneren Lumen (11) befindet; und
das Führungsdrahtlumen (22) eine Katheterseitenwandöffnung (21) aufweist, die ein Stück von dem dehnbaren Element entfernt aus dem Lumen ausgeschnitten ist, wobei sich die Katheterseitenwandöffnung (21) in Fluidverbindung mit dem inneren Führungsdrahtlumen (229 und dem inneren Hüllenlumen (11) befindet, wobei die Katheterseitenwandöffnung (21) in dem Katheter und die Hüllenseitenwandöffnung (18) in der Hülle (10) so zusammenwirken, daß das Durchführungssystem schnell über den sich an der Verwendungsposition befindenden Führungsdraht (26) vorgeschoben werden kann.

2. System nach Anspruch 1, ferner gekennzeichnet durch einen Schlitz (19), der sich zwischen der distalen Hüllenendöffnung (17) und der Hüllenseitenwandöffnung (18) befindet, so daß die Hülle (10) im Verhältnis zu dem Katheter (12) axial und proximal bewegt werden kann, ohne daß die Position des Führungsdrahtes (26) verändert wird, um die Streckeinrichtung (16) an dem dehnbaren Element (14) zur Entfaltung zu dem Inneren des Körperlumens auszurichten.

3. System nach Anspruch 1 oder 2, ferner gekennzeichnet durch einen Schlitz (23), der sich zwischen der Katheterendöffnung (20) und der Katheterseitenwandöfnung (21) befindet.

4. System nach einem der vorstehenden Ansprüche, ferner dadurch gekennzeichnet, daß die Einstellungseinrichtung einen Manipulator (15) aufweist, der folgendes umfaßt:
ein elongiertes Gehäuse (31) mit proximalen und distalen Enden sowie mit einer Innenkammer;
eine Kappe (33) mit dadurch verlaufenden Gewindedurchgang, und wobei die Kappe drehbar an einem Ende des elongierten Gehäuses (31) angebracht ist; und
ein längsbewegliches Antriebselement (34) mit Außengewinde, wobei sich das Element mindestens teilweise in der Innenkammer des elongierten Gehäuses befindet und ferner mit einem distalen Ende, das sich durch den zentralen Durchgang der Kappe (33) erstreckt, wobei eine Rotation der Kappe eine axiale Bewegung des Antriebselements (34) bewirkt.

5. System nach Anspruch 4, ferner dadurch gekennzeichnet, daß das längsbewegliche Antriebselement (34) einen zentralen Durchgang aufweist, der das proximale Ende des Katheters (12) aufnehmen kann.

6. System nach Anspruch 5, ferner dadurch gekennzeichnet, daß das proximale Ende des Katheters (12) an dem Manipulatorgehäuse (31) befestigt ist.

7. System nach Anspruch 5 oder 6, ferner dadurch gekennzeichnet, daß die Hülle (10) an dem distalen Ende des längsbeweglichen Antriebselements (34) befestigt ist, das sich aus dem distalen Ende des Manipulators (15) erstreckt.

8. System nach einem der vorstehenden Ansprüche, ferner gekennzeichnet durch eine dehnbare Streckeinrichtung (16), die um die Außenseite des dehnbaren Elements (14) angebracht ist.

## Revendications

1. Système permettant la distribution rapide par un orifice corporel de patient d'un élargisseur dilatable sur un fil de guidage en place (26), le système comportant une gaine allongée (10) avec des extrémités proximale et distale, qui protège l'élargisseur au cours de la distribution et qui est enlevable à distance de l'élargisseur pour permettre le déploiement de celui-ci, un premier orifice intérieur (11) s'étendant entre l'extrémité proximale et l'extrémité distale de la gaine (10), l'orifice et la gaine définissant une ouverture d'extrémité de gaine (17) dans l'extrémité distale, l'ouverture (17) étant en communication pour un fluide avec le premier orifice intérieur (11), un cathéter allongé (12) avec des extrémités proximale et distale, agencé à l'intérieur du premier orifice intérieur (11) de la gaine (10), le cathéter comportant un élément dilatable (14) proximalement adjacent à son extrémité distale, l'élément dilatable (14) étant adapté pour recevoir autour de sa partie extérieure un élargisseur dilatable (16), le cathéter comportant de plus un orifice intérieur de gonflage (27) en communication pour un fluide avec l'intérieur de l'élément dilatable (14) et un orifice intérieur de fil de guidage (22) s'étendant entre un point proximal de l'extrémité proximale de l'élément dilatable à l'extrémité distale du cathéter (12), l'orifice de fil de guidage (22) et l'extrémité distale du cathéter définissant une ouverture d'extrémité de cathéter (20) en communication pour un fluide avec à la fois l'orifice intérieur de fil de guidage (22) et l'orifice intérieur de gaine (11) et un moyen (15) pour ajuster la position axiale de la gaine (10) par rapport à la position du cathéter (12), actionnable à distance du patient, le moyen d'ajustement permettant de disposer l'élargisseur (16) sur l'élément dilatable (14) à exposer à l'intérieur de l'orifice corporel, point auquel l'élargisseur peut être dilaté et déployé par gonflage de l'élément dilatable, le système précité étant caractérisé en ce que :
l'orifice (11) comprend une ouverture de paroi latérale de gaine (18) découpée dans la gaine à une distance proche de l'ouverture d'extrémité de gaine, l'ouverture de paroi latérale de gaine (18) étant en communication pour un fluide avec le premier orifice intérieur (11), et
l'orifice de fil de guidage (22) comprend une ouverture de paroi latérale de cathéter (21) découpée à une distance proche de l'élément dilatable, l'ouverture de paroi latérale de cathéter (21) étant en communication pour un fluide avec à la fois l'orifice intérieur de fil de guidage (22) et l'orifice intérieur de gaine (11), l'ouverture de paroi latérale de cathéter (21) dans le cathéter et l'ouverture de paroi latérale de gaine (18) dans la gaine (10) coopérant pour permettre de faire progresser rapidement le système de distribution sur le fil de guidage en place (26).

2. Système suivant la revendication 1, caractérisé de plus par une lente (19) qui est agencée entre l'ouverture d'extrémité distale de gaine (17) et l'ouverture de paroi latérale de gaine (18), de telle sorte que la gaine (10) puisse être déplacée axialement et proximalement par rapport au cathéter (12) sans changer la position du fil de guidage (26), de manière à exposer l'élargisseur (16) sur l'élément dilatable (14) à l'intérieur de l'orifice corporel pour être déployé.

3. Système suivant l'une ou l'autre des revendications 1 et 2, caractérisé de plus par une lente (23) qui est agencée entre l'ouverture d'extrémité de cathéter (20) et l'ouverture de paroi latérale de cathéter (21).

4. Système suivant l'une quelconque des revendications précédentes, caractérisé de plus en ce que le moyen d'ajustement comprend un manipulateur (15) comprenant :
une enveloppe allongée (31) comportant des extrémités proximale et distale et une chambre intérieure,
une capsule (33) comportant un passage fileté et qui est montée à rotation sur une extrémité de l'enveloppe allongée (31), et
un élément de commande déplaçable longitudinalement (34) comportant un extérieur fileté, qui est agencé au moins partiellement à l'intérieur de la chambre intérieure de l'enveloppe allongée, et de plus comportant une extrémité distale s'étendant dans le passage central de la capsule (33), la rotation de la capsule provoquant le déplacement axial de l'élément de commande (34).

5. Système suivant la revendication 4, caractérisé de plus en ce que l'élément de commande déplaçable longitudinalement (34) comporte un passage central adapté pour loger l'extrémité proximale du cathéter (12).

6. Système suivant la revendication 5, caractérisé de plus en ce que l'extrémité proximale du cathéter (12) est fixée à l'enveloppe de manipulateur (31).

7. Système suivant l'une ou l'autre des revendications 5 et 6, caractérisé de plus en ce que la gaine (10) est fixée à l'extrémité distale de l'élément de commande déplaçable longitudinalement (34) s'étendant hors de l'extrémité distale du manipulateur (15).

8. Système suivant l'une quelconque des revendications précédentes, caractérisé de plus par la présence d'un élargisseur dilatable (16) monté autour de la partie extérieure de l'élément dilatable (14).
